# EUROPÄISCHE PATENTSCHRIFT

(11) **EP 2 240 124 B1**
(45) Veröffentlichungstag und Bekanntmachung des Hinweises auf die Patenterteilung: **20.06.2012**
(21) Anmeldenummer: 09707950.3
(22) Anmeldetag: 05.02.2009
(51) Int. Cl.: A61F 2/64, A61F 5/01, A61F 2/68

(54) **ORTHOPÄDISCHES KNIEGELENK SOWIE VERFAHREN ZUR STEUERUNG EINES ORTHOPÄDISCHEN KNIEGELENKES**
ORTHOPEDIC KNEE JOINT AND METHOD FOR CONTROLLING AN ORTHOPEDIC KNEE JOINT
ARTICULATION DU GENOU ORTHOPÉDIQUE, ET PROCÉDÉ DE COMMANDE D'UNE ARTICULATION DU GENOU ORTHOPÉDIQUE

(30) Priorität: 07.02.2008 DE 102008008284
(43) Veröffentlichungstag der Anmeldung: 20.10.2010
(73) Patentinhaber: Otto Bock HealthCare GmbH, 37115 Duderstadt (DE)
(72) Erfinder: PUSCH, Martin, 37115 Duderstadt (DE); KAMPAS, Philipp, 1020 Wien (AT)
(74) Vertreter: Stornebel, Kai
(86) Internationale Anmeldenummer: PCT/DE2009/000162
(87) Internationale Veröffentlichungsnummer: WO 2009/097841

(56) Entgegenhaltungen:
- EP-A- 0 549 855
- WO-A-2005/037151
- WO-A-2009/059594
- DE-A1- 4 004 988

## Beschreibung

Die Erfindung betrifft ein orthopädisches Kniegelenk mit einem Oberteil, an dem obere Anschlussmittel angeordnet sind, einem schwenkbar an dem Oberteil gelagerten Unterteil mit Anschlussmitteln für prothetische Komponenten und einem Anschlag zur Begrenzung einer Extensionsbewegung sowie ein Verfahren zur Steuerung eines orthopädischen Kniegelenkes.

Ziel einer prothetischen Versorgung ist es grundsätzlich, einen möglichst guten Ersatz der verlorenen Gliedmaße bereitzustellen, möglichst ohne funktionale Einschränkungen. Muss bei einem Patient eine Oberschenkel prothese mit einem Prothesenkniegelenk vorgesehen werden, ergeben sich eine Vielzahl an konstruktiven Möglichkeiten, ein solches Prothesenkniegelenk auszugestalten.

Ein einfaches, passives, monozentrisches Prothesenkniegelenk ist in der Konstruktion und Fertigung besonders einfach, bildet jedoch das natürliche Kniegelenk nur unvollkommen nach und ermöglicht insbesondere kein natürliches Gangbild. Eine Sicherung gegen ein Wegknicken des Prothesenkniegelenkes kann über einen entsprechend stabilen Aufbau, also die Zuordnung der einzelnen Prothesenkomponenten zueinander und zu dem Körper erfolgen, gegebenenfalls unterstützt durch eine Verriegelungseinrichtung bei einem sogenannten Sperrkniegelenk. Bei einem solchen Prothesenkniegelenk wird das Knie zum Ende der Schwungphase stets vollständig gestreckt, wodurch die monozentrischen Kniegelenke wegen des Erfordernisses eines statisch sicheren Aufbaus auch bei einem Fersenauftritt keine oder nur eine geringe Neigung haben, einzubeugen. Dies führt dazu, dass die Stoßbelastung bei einem Fersenauftritt unmittelbar in den Oberschenkelstumpf oder in die Hüfte eingeleitet wird, während physiologisch gesehen bei einem Fersenauftritt eine Beugung des natürlichen Kniegelenkes um ca. 25° erfolgt, was eine deutliche Auftrittsdämpfung zur Folge hat.

Neben monozentrischen Prothesenkniegelenken sind polyzentrische Prothesenkniegelenke vorhanden, die bei entsprechend hoher Lage des Momentandrehpunktes der Strecklage nicht nur beim Stehen, sondern auch unter Fersenlast zu Beginn der Standphase des Gehens eine ausgeprägte inhärente Stabilität aufweisen, so dass das Prothesenkniegelenk in gestreckter Stellung auch ohne aufgebrachtes Hüftstreckmoment sicher ist. Ebenfalls kann mit einem solchen polyzentrischen Prothesenkniegelenk eine harmonische Überleitung in die Schwungphase und eine Einleitung einer Kniebeugung unter Vorfußlast erreicht werden. Dadurch erfolgt eine Annäherung an das natürliche Gangbild. Polyzentrische Prothesenkniegelenke weisen häufig Dämpfereinrichtungen auf, mit denen es möglicht ist, eine elastisch abgefederte oder gedämpfte Kniebeugung ohne Stabilitätsverlust einzuleiten. Ein polyzentrisches Prothesenkniegelenk mit einem einstellbaren Schwenkanschlag ist in der DE 40 04 988 A1 beschrieben.

EP 549 855 A2 beschreibt ein System zum Steuern eines Prothesenkniegelenkes mit einem hydraulischen Dämpfer, der in Flexionsrichtung und Extensionsrichtung auf der Grundlage von Sensordaten individuell verstellbar ist. Werden bestimmte Schwellwerte erreicht, werden in einer Steuereinrichtung abgelegte Dämpfungseinstellungen vorgenommen. Die Einstellung erfolgt zur Verbesserung des Schwungverhaltens des Prothesenkniegelenkes.

Die WO 2005/037151 A1 beschreibt ein polyzentrisches Kniegelenk mit einer Dämpfereinrichtung, bei der die Dämpfung in Flexionsrichtung oder Extensionsrichtung durch ein Ventil verändert werden kann. Das Ventil ist als eine drehbare Scheibe aufgebaut, die zur Verbesserung des

Schwingverhaltens den gewünschten Widerstand bereitstellen kann.

Darüber hinaus sind sogenannte aktive Prothesen kniegelenke bekannt, die mittels motorischer Unterstützung das Einleiten der Beugung und Streckung auf der Grundlage von Sensordaten bewirken. Ebenfalls sind aktive Dämpfereinrichtungen vorhanden, um die Dämpfung an die jeweiligen Bedürfnisse anzupassen. Solche Prothesenkniegelenke sind in Konstruktion und Herstellung außerordentlich aufwendig.

Ausgehend von diesem Stand der Technik liegt der Erfindung die Aufgabe zugrunde, ein preiswertes Prothesenkniegelenk und ein Verfahren zur Steuerung eines Prothesenkniegelenkes bereitzustellen, mit denen dem Nutzer ein angenehmes Gehen und entspanntes Stehen ermöglicht werden kann.

Erfindungsgemäß wird diese Aufgabe durch ein orthopädisches Kniegelenk mit den Merkmalen des Anspruchs 1 sowie ein Verfahren zur Steuerung des orthopädischen Kniegelenkes mit den Merkmalen des Anspruchs 6 gelöst. Vorteilhafte Ausgestaltungen und Weiterbildungen der Erfindung sind in den jeweiligen Unteransprüchen beschrieben.

Das erfindungsgemäße orthopädisches Kniegelenk mit einem Oberteil, an dem obere Anschlussmittel angeordnet sind, einem verschwenkbar an dem Oberteil gelagerten Unterteil, mit Anschlussraitteln für prothetische Komponenten, und einem Anschlag zur Begrenzung einer Extensionsbewegung sieht vor, dass der Anschlag verlagerbar ausgebildet und mit einer Verstelleinrichtung gekoppelt ist, die wiederum mit einer Steureinrichtung gekoppelt ist, die in Abhängigkeit von Sensordaten die Verstelleinrichtung aktuiert und die Position des Anschlages verändert. Unter einem orthopädischen Kniegelenk werden sowohl Prothesenkniegelenke als auch Ortheswkniegelenh verstanden. Wird nachfolgend von Kniegelenke gesprochen, sind Orthesen- und Prothesenkniegelenke gemeint, natürliche Kniegelenke werden gesondert als solche bezeichnet. Wird bei einem Kniegelenk, insbesondere bei einem Einachs-Kniegelenk, eine verstellbare Extensionsanschlagposition vorgesehen, wird für das Gehen der Extensionsanschlag worverlagert, wodurch sich eine größere Neigung bei einem Fersenauftritt zur Beugung des Kniegelenkes und damit eine erhöhte Dämpfung beim Einleiten der Standphase realisieren lässt. Für das Stehen wird die Verstelleinrichtung dergestalt aktiviert, dass der Extensionsanschlag zurückgenommen wird, so dass sich ein statisch sichererer Orthesen- oder Prothesenaufbau realisieren lässt. Durch die aktive Verstellung, der wirksamen Position des Extensionsanschlages kann dem Nutzer dadurch ein angenehmes Gehen durch die dynamisierende Vorverlagerung und die Auftrittsdämpfung und ein entspanntes Stehen aufgrund eines sicheren Aufbaus bereitgestellt weiden.

Vorteilhafterweise sieht die Verstelleinrichtung einen Elektromotor vor, beispielsweise einen Schrittmotor, der durch eine entsprechende Ansteuerung eine hochgenaue Positionierung des Anschlages in Abhängigkeit von den Sensordaten realisieren lässt,

Alternativ dazu kann die Verstelleinrichtung den Anschlag hydraulisch verstellen, wobei aufgrund der grundsätzlichen Dämpfungseigenschaften eines hydraulischen Verstellsystem gleichzeitig eine Anschlagdämpfung realisiert wird. Bei einer rein elektromotorischen Verstellung kann der Anschlag ebenfalls mit einer Anschlagdampfung ausgerüstet sein, um sowohl die mechanischen Komponenten des Kniegelenkes als auch den Oberschenkelstumpf zu entlasten.

Die Sensordaten werden bevorzugt über Sensoren ermittelt, die als Beugewinkelsensor, Neigungssensor, Beschleunigungssensor und/oder Kraftsensor ausgebildet sein können, damit die für die Detektion des jeweiligen Bewegungszustandes erforderlichen Daten mit einer möglichst hohen Genauigkeit und Verfügbarkeit bereitgestellt werden können.

Der Anschlag kann über ein Gewinde mit der Verstelleinrichtring gekoppelt sein, so dass eine Spindelverstellung erfolgen kann, wobei entweder die Spindel selbst oder die Spindelmutter, also das Innengewinde oder das Außengewinde angetrieben werden kann, wobei sich das jeweils nicht unmittelbar angetriebene Element in die entsprechende Richtung verlagert.

Das erfindungsgemäße Verfahren zur Steuerung eines orthopädischen Kniegelenkes mit einem Extensionsanschlag und einer Verstelleinrichtung, mit der die Position des Extensionsanschlages verändert werden kann, sieht vor, dass die Position des Extensionsanschlages in Abhängigkeit von Sensordaten, insbosondere bezüglich der Gehgeschwindigkeit und/oder der Schrittweite verändert wird, wobei der Extensionsanschlag für das Gehen vorverlagert und für das Stehen zurückgenommen wird. Die Gehgeschwindigkeit und/oder die Schrittweite wird dabei über Sensoren ermittelt, wobei die Einstellroutine bzw. Steuerungsroutine vorsieht dass bei einer Schrittgeschwindigkeit von 0, also beim Stehen, eine maximale Extension eingestellt wird, um einen möglichst sicheren Aufbau bereitstellen.

Um möglichst wenig Energie zu verbrauchen und ein gleichmäßiges Gangbild zu erreichen, wird die Veränderung der Position des Extensionsanschlages während der Schwungphase durchgeführt, vorzugsweise wird eine kontinuierliche Anpassung der Position des Endanschlages durchgeführt, was dazu führt, dass auch Veränderungen in der Gehgeschwindigkeit bzw. in der Schrittweite Rechnung getragen wird. Damit ist es möglich, dass der stabile Aufbau, der beim Stehen bevorzugt wird, beim Gehen so verändert wird, dass ein standphasendynamisches Kniegelenk auch bei höheren Gehgeschwindigkeiten zur Auftrittsdämpfung beitragen kann.

Die Veränderung der Position des Streckanschlages erfolgt unabhängig von einer eventuellen Veränderung des Bewegungswiderstandes in Flexions- oder Extensionsrichtung. Der Streckanschlag kann neben einer Verstellung rein mechanischer Anschläge, die in Gestalt von Anschlagschultern, Bolzen oder dergleichen ausgebildet sein können, auch durch das Schließen von Ventilen erfolgen. Die Kniegelenke, die eine verstellbare Dämpfereinrichtung aufweisen, können dahingehend ausgebildet sein, dass die Steuereinrichtung in Abhängigkeit der ermittelten Sensordaten das Extensionsdämpfungsventil in einer bestimmten Stellung des Unterteiles relativ zu dem Oberteil schließt, um dadurch eine Begrenzung der Verschwenkbewegung zu erreichen. Das Ventil ist bevorzugt als Sperrventil ausgebildet, das bei Erreichen einer vorgegebenen Stellung geschlossen wird, ggf. kann eine verstellbare Drossel, die bei der Anpassung der Extensionsdämpfung eingesetzt wird, als Absperrventil eingesetzt werden.

Nachfolgend werden Ausführungsbeispiele der Erfindung anhand der beigefügte Figuren näher erläutert. Gleiche Bezugsziffern in den Figuren bezeichnen gleiche Komponenten. Es zeigen:
- Figur 1 -: eine schematische Darstellung einer Oberschenkelprothese in leicht gebeugter Stellung;
- Figur 2-: eine Oberschenkelprothese in vollständig gestreckter Stellung;
- Figur 3 -: eine Oberschenkelprothese mit einer längenveränderlichen Steuereinheit;
- Figur 4 -: eine Oberschenkelprothese gemäß Figur 3 mit einer motorisch verstellbaren Steuereinheit;
- Figur 5 -: eine Oberschenkelprothese mit einem längenveränderlichen Koppelelement;
- Figur 6 -: eine Oberschenkelprothese mit einem motorisch längenveränderlichen Koppelelement;
- Figur 7 -: eine Oberschenkelprothese mit einem in einer Steuereinheit verschieblich gelagertem Koppelelement; sowie
- Figur 8 -: eine Oberschenkelprothese mit einem motorisch verstellbaren mechanischen Anschlag.

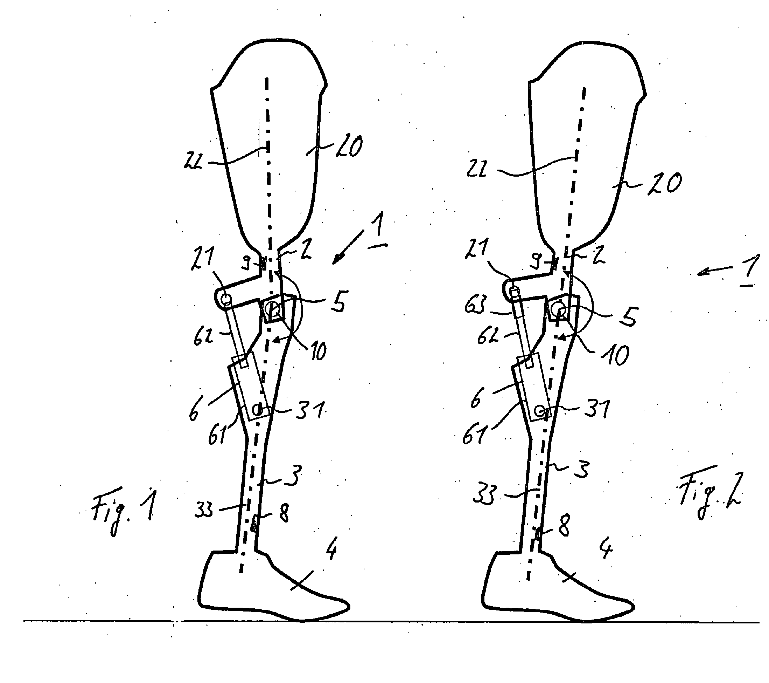

Figur 1 zeigt einen grundsätzlichen Aufbau eines orthopädischen Kniegelenkes in Gestalt einer Oberschenkelprothese 1 mit einem Oberteil 2 und einem Unterteil 3, n dem ein Prothesenfuß 4 befestigt ist. Das Oberteil 2 und das Unterteil 3 sind schwenkbar um eine Schwenkachse 5 miteinander verbunden. An dem proximalen Ende des Oberteils 2 ist ein oberes Anschlussmittel 20 in Gestalt eines Prothesenschaftes angeordnet. Bei einer Ausbildung des orthopädischen Kniegelenkes als eine Orthese sind die Anschlussmittel in Gestalt einer den Oberschenkel teilweise umgebenden Schale oder in Gestalt von Riemen oder anderen Befestigungsmitteln ausgebildet. Das Oberteil 2 und das Unterteil 3 werden dann medial oder lateral von dem natürlichen Bein geführt, die orthopädische Komponente 4 ist dann eine Fußschale, in die der natürliche Fuß eingesetzt wird.

Zwischen dem Oberteil 2 und dem Unterteil 3 ist eine Steuereinheit 6 angeordnet, die einen oberen Befestigungspunkt 21 und einen unteren Befestigungspunkt 31 aufweist. Der obere Befestigungspunkt ist dem Oberteil 2 zugeordnet, während der untere Befestigungspunkt 31 dem Unterteil 3 zugeordnet ist. Die Steuereinheit 6 kann gelenkig an den Befestigungspunkten 21, 31 gelagert sein.

Die Steuereinheit 6 weist in dem vorliegenden Beispiel einen Zylinder 61 und eine Kolbenstange 62 auf, die verschiebbar zueinander gelagert sind. Die Kolbenstange 62 ist an dem oberen Befestigungspunkt 21 angeordnet, während der Zylinder 61 an dem unteren Befestigungspunkt 31 angeordnet ist. Wird das Unterteil 3 relativ zu dem Oberteil 2 verschwenkt, beispielsweise gebeugt, fährt die Kolbenstange 62 in den Zylinder 61 ein, bei einer Streck- oder Extensionsbewegung fahren die Komponenten 61, 62 auseinander. Innerhalb der Steuereinheit 6 kann das Maß eingestellt werden, wie weit die Kolbenstange 62 aus dem Zylinder 61 herausfahren kann. In der dargestellten Position ist das Kniegelenk maximal gestreckt, wobei die Längsachsen 22, 33 des Oberteils 2 und des Unterteils 3 in einem Winkel zueinander stehen, der von 180° abweicht. Im vorliegenden Fall ist das Oberteil 2 zu dem Unterteil 3 leicht gebeugt, trotz vollständig gestreckter Prothese, die an dem Anschlag anliegt, der von der Kolbenstange 62 in dem Zylinder 61 ausgebildet wird.

Sowohl an dem Unterteil 3 als auch an dem Oberteil 2 sind Sensoren 8, 9 angeordnet, die innerhalb der Prothese 1 auftretenden Momente, Kräfte oder Beschleunigungen messen können. Ebenfalls ist es vorgesehen, dass diese Sensoren 8, 9 Informationen über die Neigung des Oberteils 2 oder des Unterteils 3 entweder zueinander oder absolut ermitteln. Ebenfalls ist innerhalb des Kniegelenks 1 ein Beugewinkelsensor 10 im Bereich der Drehachse 5 angeordnet, um die Winkelstellung der Achsen 22, 23 zueinander und damit des Oberteils 2 zu dem Unterteil 3 zu ermitteln. Diese Sensoren 8, 9, 10 sind mit der Steuereinrichtung 6 gekoppelt, so dass auf Grundlage der Sensordaten über eine nicht dargestellte Verstelleinrichtung das Maß der Extension beeinflusst werden kann, indem die Ausfahrlänge der Kolbenstange 62 variiert wird. In der Steuereinrichtung 6 sind entsprechende Datenverarbeitungsmittel vorgesehen, die auf der Grundlage der vorhandenen Daten die Position des Anschlages und den Verstellweg berechnen.

In der Figur 2 ist ein Kniegelenk 1 gezeigt, das im Wesentlichen dem der Figur 1 entspricht. Die Kolbentange 62 ist dabei mit einer Hülse 63 versehen, die verschieblich oder verdrehbar an der Kolbenstange 62 gelagert ist und über die eine Längeneinstellung erfolgen kann. Wird beispielsweise die Kolbenstange 62 über eine Verstelleinrichtung verdreht, kann diese sich in die Hülse 63, die mit einem Gewinde versehen ist, hinein- oder hinausdrehen, um bei einem gleichbleibenden Endanschlag innerhalb des Zylinders 61 eine Variation des Kniewinkels zu realisieren. In der dargestellten Ausführungsform der Figur 2 ist das Kniegelenk 1 in einer vollständig gestreckten Stellung gezeigt, das heißt, dass die Längsachsen 22, 33 in einem Winkel von 180° zueinander stehen, was die bevorzugte Stellung während des Stehens darstellt, da hierdurch ein stabiler Aufbau erreicht wird.

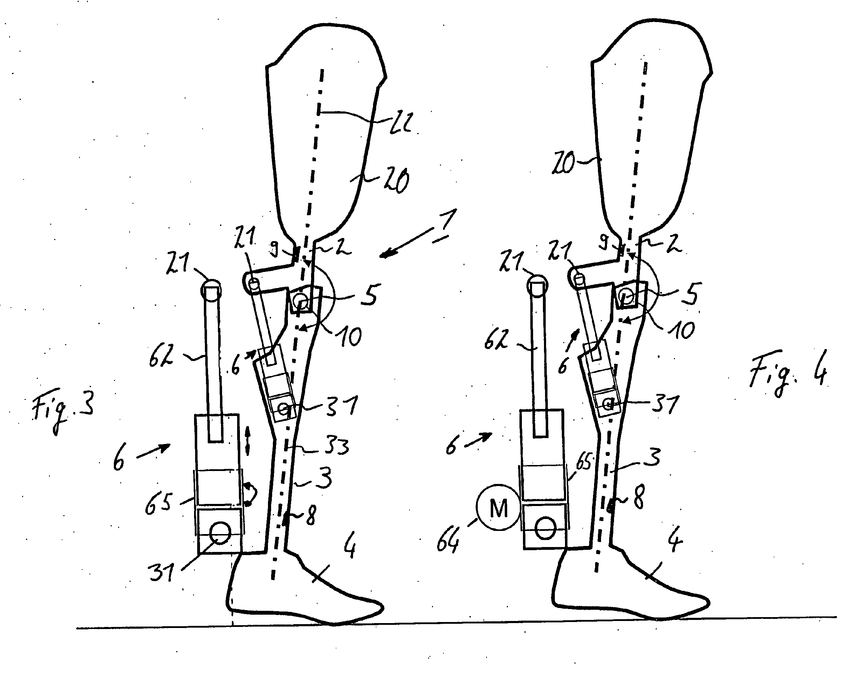

Die in der Figur 3 gezeigte Prothese unterscheidet sich von der in der Figur 1 gezeigten dadurch, dass die Steuereinheit 6 in ihrer Länge veränderlich ausgebildet ist, beispielsweise über eine Schraubhülse 65 und gegensinnig zueinander ausgebildete Gewindeeinrichtungen, so dass durch Drehung der Schraubhülse 65, die in vergrößerter Darstellung links neben dem Kniegelenk 1 gezeigt ist, ein Teil der Steuereinrichtung 6 in Richtung auf den oberen Befestigungspunkt 21 verfahren oder von diesem entfernt werden kann, wie durch den Doppelpfeil angedeutet ist. Dies geschieht durch ein entsprechendes Verdrehen der Schraubhülse 65, so dass über die Veränderung der Länge der Steuereinheit 6 eine Verlagerung der Anschlagposition bewirkt wird, was wiederum zu einer Veränderung des maximalen Kniewinkels in der gestreckten Position führt. Ein Teil der Steuereinheit 6, der mit dem unteren Befestigungspunkt 31 gekoppelt ist, bleibt dabei ortsfest.

In der Figur 4 ist dargestellt, dass diese Verlagerung elektromotorisch über einen Antriebsmotor 64, der die Verstelleinrichtung darstellt, bewirkt werden kann. Die Verstellung erfolgt dann aufgrund einer Berechnung innerhalb der Steuereinheit 6, die auf Grundlage der von den Sensoren 8 bis 10 gelieferten Daten durchgeführt wird.

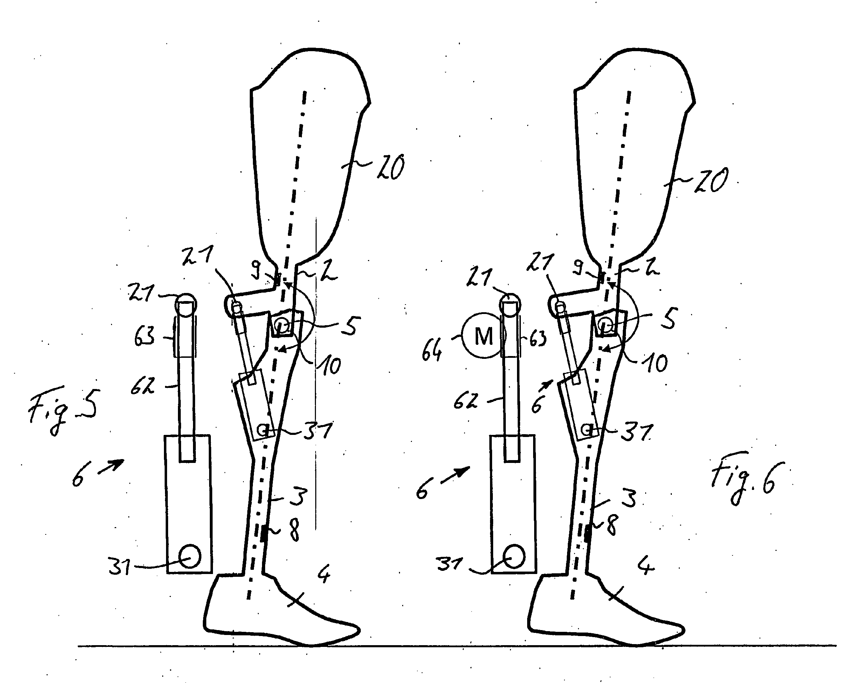

In der Figur 5 ist eine Ausgestaltung gemäß der Figur 2 dargestellt, zusammen mit einer vergrößerten Darstellung der Steuereinheit 6. Die Schraubhülse 65 kann die Länge der Kolbenstange 62, also eines Koppelelementes zwischen dem Unterteil 3 und dem Oberteil 2, bewirken. Auch hier ist der Anschlag in der Steuereinheit 6 realisiert, so dass bei einer Veränderung der Länge von Koppelelementen zwischen dem Oberteil 2 und dem Unterteil 3 eine Veränderung der Anschlagsposition erfolgen kann. In der Figur 6 ist gezeigt, dass diese Veränderung der Länge der Koppelemente, vorliegend der Kolbenstange 62, über einen Elektromotor 64 erfolgt. Auch hier kann die Verstellung über eine drehbare Gewindestange oder eine drehbare Gewindehülse 63, die in einem Außengewinde der Kolbenstange 62 eingreift, erfolgen.

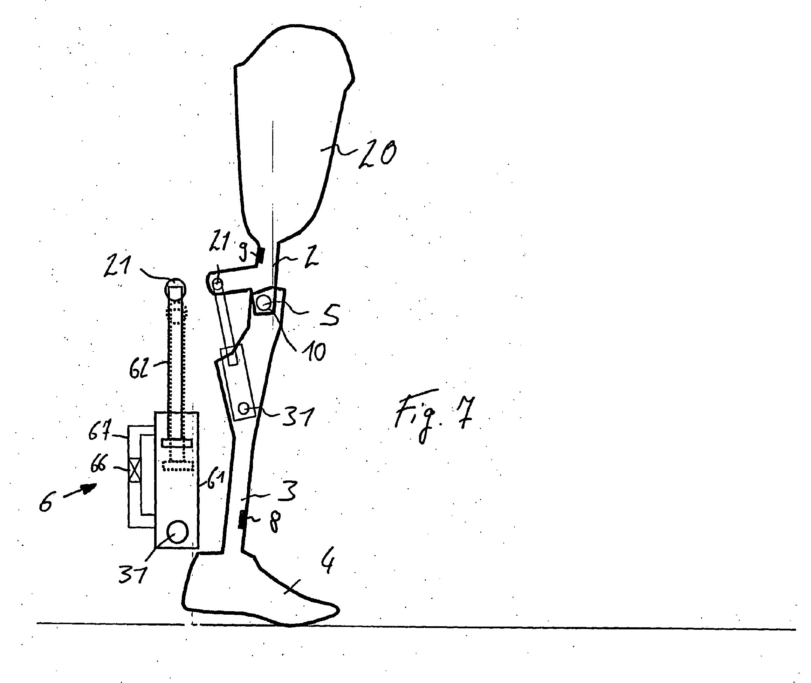

In der Figur 7 ist eine Variante der Veränderung des Anschlages gezeigt. Auch hier wird der Anschlag in der Steuereinrichtung 6 realisiert, indem die maximale Ausfahrlänge der Kolbenstange 62 verändert wird. Dies erfolgt durch eine Verschiebung der Kolbenstange 62 bzw. des Koppelelementes 62 in Richtung auf den unteren Befestigungspunkt 31 in den Zylinder 61, beispielsweise indem ein Ventil 66 innerhalb einer Bypassleitung 67 geöffnet wird, so dass ein Hydraulikfluid in eine obere Zylinderkammer eindringen kann, so dass die Kolbenstange 62 weiter in den Zylinder 61 eintauchen kann. Gleichzeitig wird die Anschlagsposition in Extensionsrichtung verändert, da die Kolbenstange 62 frühzeitiger gegen den maximalen Anschlag innerhalb des Zylinders 61 anstößt bzw. gegen die Hydraulikflüssigkeit drückt. Somit erfolgt durch Verschieben des Koppelgliedes bzw. der Kolbenstange 62 in der Steuereinheit 6 eine Begrenzung zu geänderten Kniewinkeln bei einem vollständig gestreckten Kniegelenk 1. Ein vollständig gestrecktes Kniegelenk liegt dann vor, wenn das Unterteil 3 nicht mehr weiter extendiert werden kann, auch wenn noch keine Winkelstellung von 180° erreicht ist.

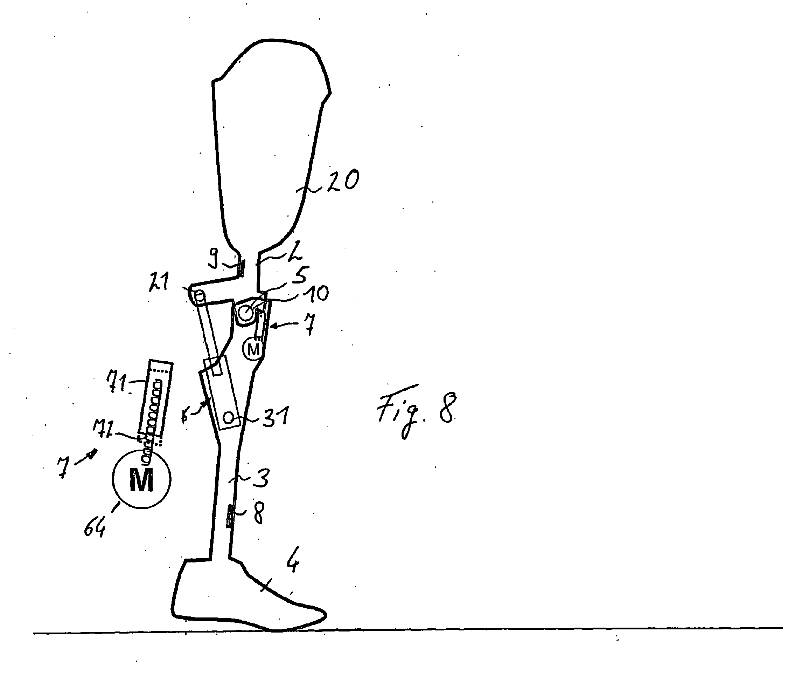

Eine alternative Ausführungsform ist in der Figur 8 dargestellt, bei der der Anschlag unabhängig von der in der Regel als hydraulische Dämpfereinheit ausgebildeten Steuereinheit 7 ausgebildet ist. Der separate Anschlag 7 führt über eine motorische Verlagerung einer Hülse dazu, dass eine Endstreckung bei unterschiedlichen Kniewinkeln erreicht wird. Die Steuersignale des Elektromotors 64, der die Verstelleinrichtung ausbildet, werden von der Steuereinrichtung 6 geliefert, die mit den Sensoren 8 bis 10 gekoppelt ist. Je nach ermittelten Sensordaten, beispielsweise Neigung des Oberteils 2 oder des Unterteils 3, Axialbelastung durch Auftreten des Prothesen- oder Orthesenfußes 4 oder des Kniewinkels über den Beugewinkelsensor 6, werden unterschiedliche Anschlagspositionen eingestellt, so dass eine verbesserte Anpassung an das normale Gangbild erzielt werden kann. In der Figur 8 erfolgt eine Verstellung der Anschlagshülse 71 und des Anschlages 7 insgesamt über ein Gewinde 72, das mit dem Elektromotor 64 gekoppelt ist.

Die zu den im Ausführungsbeispiel gezeigten Prothesenkniegelenke 1 gemachten Ausführungen gelten entsprechend auch für Orthesenkniegelenke, deren maximaler Extensionswinkel ebenfalls in Abhängigkeit von Sensordaten verändert werden kann.

Neben der dargestellten Ausfuhrungsform einer Verstelleinrichtung 64 als ein Elektromotor kann auch eine hydraulische Anschlagverstellung, wie sie in der Figur 7 angedeutet ist, erfolgen. Die Aktuierung des Ventils 66 kann hydraulisch oder elektromechanisch erfolgen, beispielsweise über einen Schalter oder ein schaltbares Ventil. Ebenfalls können weitere Sensoreinrichtungen vorgesehen sein, die über die genannten Sensordaten hinaus weitere Daten der Steuereinrichtung zufuhren. Innerhalb der Steuereinrichtung 6, die gleichzeitig als eine Dämpfereinrichtung ausgebildet ist, sind Recheneinrichtungen vorhanden, die eine entsprechende Verstellung der Anschlagsposition errechnen und Steuerdaten für die Verstelleinrichtung ausgeben.

## Patentansprüche

1. Orthopädisches Kniegelenk mit einem Oberteil (2), an dem obere Anschlussmittel (20) angeordnet sind, einem verschwenkbar an dem Oberteil (2) gelagerten Unterteil (3), mit Anschfussmitteln für orthopädische Komponenten (4), und einem Anschlag (7) zur Begrenzung einer Extensionsbewegung, **dadurch gekennzeichnet, dass** der Anschlag (7) verlagerbar ausgebildet und mit einer Verstelleinrichtung (64) gekoppelt ist, die mit einer Steuereinrichtung (6) gekoppelt ist, die in Abhängigkeit von Sensordaten die Verstelleinrichtung (64) aktuiert und die Position des Anschlages (7) dahin verändert, dass ein Extensionsanschlag für das Gehen vorverlagert und für das Stehen zurückgenommen wird.

2. Orthopädisches Kniegelenk nach Anspruch 1, **dadurch gekennzeichnet, dass** die Verstelleinrichtung (64) ein Elektromotor ist.

3. Orthopädisches Kniegelenk nach Anspruch 1, **dadurch gekennzeichnet, dass** die Verstelleinrichtung (64) den Anschlag (7) hydraulisch verstellt.

4. Orthopädisches Kniegelenk nach einem der voranstehenden Ansprüche, **dadurch gekennzeichnet, dass** die Sensordaten über Sensoren (8, 9) ermittelt werden, die als Beugewinkelsensor, Beschleunigungssensor, Neigungssensor und/oder Kraftsensor ausgebildet sind.

5. Orthopädisches Kniegelenk nach einem der voranstehenden Ansprüche, **dadurch gekennzeichnet, dass** der Anschlag (7) über ein Gewinde (72) mit der Verstelleinrichtung (64) gekoppelt ist.

6. Verfahren zur Steuerung eines orthopädischen Kniegelenks mit einem Extensionsanschlag und einer Verstelleinrichtung, mit der die Position des Extensionsanschlages verändert werden kann, **dadurch gekennzeichnet, dass** die Position des Extensionsanschlages in Abhängigkeit von Sensordaten verändert wird, wobei der Extensionsanschlag für das Gehen vorverlagert und für das Stehen zurückgenommen wird.

7. Verfahren nach Anspruch 6, **dadurch gekennzeichnet, dass** aus den Sensordaten die Gehgeschwindigkeit und/oder Schrittweite ermittelt und die Position des Extensionsanschlages in Abhängigkeit davon verändert wird.

8. Verfahren nach Anspruch 6 oder 7, **dadurch gekennzeichnet, dass** bei einer Schrittgeschwindigkeit von Null eine maximale Extension eingestellt wird.

9. Verfahren nach einem der Ansprüche 6 bis 8, **dadurch gekennzeichnet, dass** die Veränderung der Position des Extensionsanschlages während der Schwungphase durchgeführt wird.

10. Verfahren nach einem der Ansprüche 6 bis 9, **dadurch gekennzeichnet, dass** eine kontinuierliche Anpassung der Position des Extensionsanschlages durchgeführt wird.

11. Verfahren nach einem der Ansprüche 6 bis 10, **dadurch gekennzeichnet, dass** der Extensionsanschlag durch das Schließen einer Sperreinrichtung in einer Hydraulikleitung verstellt wird.

## Claims

1. Orthopedic knee joint, with an upper part (2) on which upper connecting means (20) are arranged, with a lower part (3) which is mounted pivotably on the upper part (2) and has connecting means for orthopedic components (4), and with a stop (7) for limiting an extension movement, **characterized in that** the stop (7) is designed to be movable and is coupled to an adjusting device (64), which is coupled to a control device (6) that actuates the adjusting device (64) as a function of sensor data and changes the position of the stop (7) in such a way that an extension stop is shifted forward for walking and is reversed for standing.

2. Orthopedic knee joint as claimed in claim 1, **characterized in that** the adjusting device (64) is an electric motor.

3. Orthopedic knee joint as claimed in claim 1, **characterized in that** the adjusting device (64) adjusts the stop (7) hydraulically.

4. Orthopedic knee joint as claimed in one of the preceding claims, **characterized in that** the sensor data are determined via sensors (8, 9) designed as flexion angle sensor, acceleration sensor, inclination sensor and/or force sensor.

5. Orthopedic knee joint as claimed in one of the preceding claims, **characterized in that** the stop (7) is coupled to the adjusting device (64) via a thread (72).

6. Method for controlling an orthopedic knee joint with an extension stop and with an adjusting device by which the position of the extension stop can be changed, **characterized in that** the position of the extension stop is changed as a function of sensor data, wherein the extension stop is shifted forward for walking and is reversed for standing.

7. Method as claimed in claim 6 **characterized in that** the walking speed and/or stride is determined from the sensor data, and the position of the extension stop is changed as a function thereof.

8. Method as claimed in claim 6 or 7 **characterized in that**, at a walking speed of zero, a maximum extension is adopted.

9. Method as claimed in one of claims 6 through 8, **characterized in that** the change of the position of the extension stop is effected during the swing phase.

10. Method as claimed in one of claims 6 through 9, **characterized in that** the position of the extension stop is adapted continuously.

11. Method as claimed in one of claims 6 through 10, **characterized in that** the extension stop is adjusted by the closure of a blocking device in a hydraulic line.

## Revendications

1. Articulation orthopédique du genou comprenant une partie supérieure (2) à laquelle sont agencés des moyens de raccordement supérieurs (20), une partie inférieure (3) montée basculable sur la partie supérieure (2) et ayant des moyens de raccordement pour des composants orthopédiques (4), et une butée (7) pour limiter un mouvement d'extension, **caractérisée en ce que** la butée (7) est formée déplaçable et couplée avec un dispositif de réglage (64) qui est couplé avec un dispositif de pilotage (6) qui actionne le dispositif de réglage (64) en fonction de données capteur et change la position de la butée (7) de façon à faire avancer une butée d'extension, pour la marche, et la ramener en arrière, pour la position debout.

2. Articulation orthopédique du genou selon la revendication 1, **caractérisée en ce que** le dispositif de réglage (64) est un moteur électrique.

3. Articulation orthopédique du genou selon la revendication 1, **caractérisée en ce que** le dispositif de réglage (64) règle la butée (7) de façon hydraulique.

4. Articulation orthopédique du genou selon l'une quelconque des revendications précédentes, **caractérisée en ce que** les données capteur sont fournies par des capteurs (8, 9) qui sont formés par un capteur d'angle de flexion, un capteur d'accélération, un capteur d'inclinaison et/ou un capteur de forcé.

5. Articulation orthopédique du genou selon l'une quelconque des revendications précédentes, **caractérisée en ce que** la butée (7) est couplée au dispositif de réglage (64) par l'intermédiaire d'un filtage (72).

6. Procédé de pilotage d'une articulation orthopédique du genou comprenant une butée d'extension et un dispositif de réglage, permettant de modifier la position de la butée d'extension, **caractérisé en ce que** l'on modifie la position de la butée d'extension en fonction de données capteur, la butée d'extension étant avancée pour la marche et ramenée en arrière pour la position debout.

7. Procédé selon la revendication 6, **caractérisé en ce qu'**on détermine la vitesse de marche et/ou la longueur du pas à partir des données capteur et on modifie la position de la butée d'extension en fonction de ça.

8. Procédé selon la revendication 6 ou 7, **caractérisé en ce que** l'on règle une extension maximale lors d'une vitesse de pas de zéro.

9. Procédé selon l'une quelconque des revendications 6 à 8, **caractérisé en ce que** l'on met en oeuvre la modification de la position de la butée d'extension pendant la phase de lancée.

10. Procédé selon l'une quelconque des revendications 6 à 9, **caractérisé en ce que** l'on met en oeuvre une adaptation en continu de la position de la butée d'extension.

11. Procédé selon l'une quelconque des revendications 6 à 10, **caractérisé en ce que** l'on règle la butée d'extension par la fermeture d'un dispositif de blocage d'une conduite hydraulique.
